Europäisches Patentamt

European Patent Office

Office européen des brevets

(11) Publication number: **0 505 841 A2**

## EUROPEAN PATENT APPLICATION

(21) Application number: **92104259.4**

(22) Date of filing: **12.03.92**

(51) Int. Cl.5: **A61K  31/60**, A61K 31/165,
//(A61K31/60,31:07),
(A61K31/165,31:07)

(30) Priority: **25.03.91 US 674457**

(43) Date of publication of application:
**30.09.92 Bulletin  92/40**

(84) Designated Contracting States:
**AT BE CH DE DK ES FR GB GR IT LI LU NL PT SE**

(71) Applicant: **MILES INC.**
**One Mellon Center 500 Grant Str.**
**Pittsburgh, PA 15219-2502(US)**

(72) Inventor: **Daher, Lawrence J.**
**54652 Homeland Road**
**Elkhart, IN 46514(US)**
Inventor: **Siebert, John M.**
**1629 Jeanwood**
**Elkhart, IN 46514(US)**

(74) Representative: **Dänner, Klaus, Dr.**
**c/o Bayer AG Konzernverwaltung RP Patente Konzern**
**W-5090 Leverkusen 1 Bayerwerk(DE)**

(54) **Analgesic composition containing beta-carotene.**

(57)  The invention discloses a pharmaceutical composition useful as an analgesic and anti-inflammatory composed of an analgesic drug and ß-carotene.

EP 0 505 841 A2

## Field of the Invention

The invention relates to analgesic compositions in general and an analgesic composition containing ß-carotene in particular.

## Background of the Invention

Biologically acceptable antioxidant agents, such as the various antioxidant vitamins, are known to have effects upon some of the physiological systems and mechanisms involved in pain and inflammation. For instance, it is reported that the eicosanoid products of fatty acid metabolism, which are involved in the genesis and prolongation of pain symptomology and in the genesis of the symptoms and signs of inflammation, are also inhibited in formation by certain antioxidant agents.

O. Halevy and D. Sklan in 1986 reported that ß-carotene, retinol and alpha-tocopherol influence both the cyclooxygenase and lipoxygenase pathways of eicosanoid production. Prostaglandin and hydroxyicosatetraenoic acid (HETE) production in bovine seminal vesicles and kidney were considerably inhibited by ß-carotene and retinol, and to a lesser extent by alpha-tocopherol. Halevy, O., Sklan, D., "Inhibition of Arachidonic Acid Oxidation by Beta-Carotene, Retinol and Alpha-Tocopherol," Biochim. Biophy. Acta. 918 (1987) 304-307.

EP 0 380 367 discloses a synergistic composition comprising an antioxidant and an arylpropionic, non-steroidal anti-inflammatory drug (NSAID). Preferred antioxidants include alpha tocopherol, ascorbic acid, Vitamin A and ß carotene. The arylpropionic NSAIDs include ibuprofen, ketoprofen and naproxen. The composition is useful for treatment of periodontal disease.

## Summary of the Invention

The invention provides a composition of enhanced analgesic and anti-inflammatory properties comprising ß-carotene and an analgesic/anti-inflammatory drug chosen from the group consisting of aspirin, acetaminophen and mixtures thereof. A preferred composition is ß-carotene and aspirin.

## Description of the Invention

It has now been discovered that ß-carotene can be used to form a combination with an analgesic which combination has increased pain and inflammation reducing properties. ß-carotene is a naturally occurring compound, found abundantly in plants, and also prepared synthetically. It is used commercially as a food colorant, and a nutritional supplement. As a nutritional supplement it is commonly supplied as 15 mg softgel pharmaceutical capsule. In lower dosages it is used as a source of Vitamin A activity.

In contrast to ß-carotene, alpha-tocopherol, another biological antioxidant and vitamin, has been found to antagonize the pain relief effects of aspirin. This finding seems surprising in view of alpha-tocopherol's known suppressant effects upon eicosanoid biosynthesis. However, this finding merely points out the complexity of the antioxidant/analgesic bio-interaction.

The analgesic pharmaceutical compositions of the present invention comprise, in addition to ß-carotene, at least one active analgesic drug. Typical analgesic drugs include aspirin, or a combination of aspirin with acetaminophen. These drugs are used at their usual dosage levels or less, depending upon whether greater analgesic and anti-inflammatory activity is desired, or at reduced levels to deliver equivalent or less analgesic and anti-inflammatory activity.

Aspirin is the most preferred analgesic drug. Aspirin brings to the combinations its analgesic, antipyretic and anti-inflammatory activities, coupled with its wide spectrum of other pharmacological activities, especially its anti-blood clotting effects. In the most desirable practice of this invention, aspirin is used at its usual dosage.

Acetaminophen and aspirin may also be combined together to provide a mixed analgesic component of this invention. Such combinations have found wide acceptance of usage, especially in the over-the-counter (OTC) drug market. Aspirin may be used straight or in buffered form as per common pharmaceutical practice.

Pharmaceutical tablets, illustrative of some practices of this invention have been prepared. Aspirin has been combined with ß-carotene in a single tablet combination containing 325 mg of aspirin and 2 mg of ß-carotene. Thus a usual single adult dose of two tablets provides 650 mg of aspirin and 4 mg of ß-carotene. A two tablet, four times a day, dosage regimen, provides 2600 mg of aspirin and 16 mg of ß-carotene daily.

In general ß-carotene is most desirably included in a dosage form at levels that provide up to about 25

2

mg per day of ß-carotene. Thus depending upon the division of dosage across a day's time, the levels of ß-carotene in the dosage forms can be adjusted to provide the desired total daily dosage of ß-carotene.

The following examples disclose preferred embodiments of the invention, but do not limit the applicability of the invention which is solely defined by the claims.

EXAMPLES

Pharmacological Testing

Anti-Inflammatory

The test substance was administered to 8 overnight fasted rats orally (mg/kg) one hour before the right hind paw intraplantar injection of carrageenan (0.1 mL, 1% suspension). Inhibition of paw edema by more than 30 percent (>30) three hours after carrageenan administration indicates acute anti-inflammatory activity. [Winter, C.A., Risley, E.A., and Nuss, G.W., Proc. Exper. Biol. Med., 111:544-547, 1962.]

Phenylquinone (PQ) Writhing

The test sample is administered orally (mg/kg) to a group of 8 mice one hour before injection of PQ (2 mg/kg intraperitonilly). Greater than 50 percent (>50) inhibition of the number of writhes per group of animals observed during 5 to 10 minutes after PQ, relative to a vehicle treated group, indicates possible analgesic activity. [Irwin, Samuel, Psychopharmacologia, 13:22-257, 1968.]

Example 1 - Pharmacological Test Showing Enhanced Anti-Inflammatory Action of ß-Carotene and Aspirin Combinations

The unexpected enhancement of anti-inflammatory effect of the addition of ß-carotene to aspirin is evidenced by tests conducted on rats by Panlabs Inc., 11804 North Creek Parkway South, Bothell, WA 98011. All rats were dosed orally by gavage with ß-carotene and/or aspirin. Testing was conducted according to statistical design. The following treatments were examined:

| | |
|---|---|
| $A_0$ = Aspirin vehicle | $B_0$ = ß-carotene vehicle |
| $A_1$ = Aspirin 10 mg/kg | $B_1$ = ß-carotene 10 mg/kg |
| $A_2$ = Aspirin 30 mg/kg | $B_2$ = ß-carotene 30 mg/kg |
| $A_3$ = Aspirin 100 mg/kg | $B_3$ = ß-carotene 100 mg/kg |
| $A_4$ = Aspirin 300 mg/kg | $B_4$ = ß-carotene 300 mg/kg |

The study design is shown in the following table. The abbreviation "p.o" in the table means "per os" or oral dosing.

Study Design:

A1-4 + B0 p.o. / 1 hr.    carrigeenan    interplantar injection / 3 hrs.    anti-inflammatory activity

A0 + B1-4

A1-4 + B1-4

n = 8/treatment

Results:

Mean Paw Volumes and (p-values*)

| ß-Carotene Levels | Aspirin Levels | | | | |
|---|---|---|---|---|---|
| | $A_0$ (veh.) | $A_1$ (10 mg/kg) | $A_2$ (30 mg/kg) | $A_3$ (100 mg/kg) | $A_4$ (300 mg/kg) |
| $B_0$ (veh.) | 84.0 | 79.1 | 73.9 | 58.9 | 38.8 |
| $B_1$ (10 mg/kg) | 86.0(.24) | 75.5(.03) | 73.0(.60) | 57.4(.37) | 37.0(.30) |
| $B_2$ (30 mg/kg) | 86.5(.14) | 77.4(.30) | 68.0(<.01) | 57.3(.33) | 37.0(.33) |
| $B_3$ (100 mg/kg) | 81.6(.16) | 77.4(.30) | 71.4(.14) | 53.8(<.01) | 40.4(.30) |
| $B_4$ (300 mg/kg) | 81.9(.21) | 75.6(.04) | 70.6(.05) | 55.4(.04) | 42.0(.05) |

*p-value is for comparison with zero level ß-Carotene within the same aspirin level.
Paw Volumes are in ml. x $10^{-2}$.

Statistical analysis indicates that the net response of the combination is an enhancement of anti-inflammatory activity. However, the ratio of aspirin to ß-carotene used in humans may be different than that used in the rat testing.

Example 2 - Pharmacological Test Showing Antagonism to Analgesia of Aspirin by Alpha-Tocopherol

A test was conducted using alpha-tocopherol and aspirin. The result of that test showed an antagonism to the analgesia of aspirin by alpha-tocopherol. All dosing was done orally (PO). The results of that test are given below.

| | |
|---|---|
| $A_0$ = Aspirin vehicle | $E_0$ = Alpha-tocopherol vehicle |
| $A_1$ = Aspirin $ED_{20}$ = 8.3 mg/kg | $E_1$ = Alpha-tocopherol = 300mg/kg |
| $A_2$ = Aspirin $ED_{50}$ = 25 mg/kg | $E_2$ = Alpha-tocopherol = 100mg/kg |
| $A_3$ = Aspirin $ED_{80}$ = 75 mg/kg | $E_3$ = Alpha-tocopherol = 30 mg/kg |
| | $E_4$ = Alpha-tocopherol = 10 mg/kg |
| | $E_5$ = Alpha-tocopherol = 3 mg/kg |

Test design and results:

$A_{1-3}$ + $E_0$
$A_0$ + $E_{1-5}$
$A_{1-3}$ + $E_{1-5}$

| COMPOUND | ROUTE | P.Q. WRITHING (INH. %) $\overline{X} \pm$ SEM | NOTE |
|---|---|---|---|
| $A_1$ + $E_0$ | PO | 20 ± 1 | n = 8 |
| $A_2$ + $E_0$ | PO | 48 ± 2 | n = 8 |
| $A_3$ + $E_0$ | PO | 85 ± 2 | n = 8 |
| $A_0$ + $E_1$ | PO | 3 ± 1 | n = 8 |
| $A_0$ + $E_2$ | PO | 1 ± 1 | n = 8 |
| $A_0$ + $E_3$ | PO | 3 ± 1 | n = 8 |
| $A_0$ + $E_4$ | PO | 2 ± l | n = 8 |
| $A_0$ + $E_5$ | PO | 3 ± 1 | n = 8 |
| $A_1$ + $E_1$ | PO | 13 ± 2 | n = 8 |
| $A_1$ + $E_2$ | PO | 18 ± 1 | n = 8 |
| $A_1$ + $E_3$ | PO | 18 ± 2 | n = 8 |
| $A_1$ + $E_4$ | PO | 19 ± 2 | n = 8 |
| $A_1$ + $E_5$ | PO | 20 ± 1 | n = 8 |
| $A_2$ + $E_1$ | PO | 39 ± 2 | n = 8 |
| $A_2$ + $E_2$ | PO | 40 ± 1 | n = 8 |
| $A_2$ + $E_3$ | PO | 41 ± 1 | n = 8 |
| $A_2$ + $E_4$ | PO | 41 ± 2 | n = 8 |
| $A_2$ + $E_5$ | PO | 43 ± 2 | n = 8 |
| $A_3$ + $E_1$ | PO | 75 ± 2 | n = 8 |
| $A_3$ + $E_2$ | PO | 76 ± 1 | n = 8 |
| $A_3$ + $E_3$ | PO | 78 ± 2 | n = 8 |
| $A_3$ + $E_4$ | PO | 80 ± 2 | n = 8 |
| $A_3$ + $E_5$ | PO | 81 ± 1 | n = 8 |

Statistical Analysis:

An analysis was done for aspirin and Vitamin E. The following table shows the means corresponding to the levels of aspirin and Vitamin E used. Again the writhing response was set at zero for the two-vehicle combination.

Effect of Aspirin

| Level of Vitamin E | Veh. | ED20 | ED50 | ED80 |
|---|---|---|---|---|
| Vehicle | 0 | 20 | 48 | 85 |
| 100 mg. | 1 | 18 | 40 | 76 |
| 300 mg. | 2 | 13 | 39 | 75 |

There is a statistically significant dose response effect for both aspirin and Vitamin E (p. <.001). There is also a significant antagonistic effect of Vitamin E on this response (p. <.001), as given by the significant interaction term.

Therefore, alpha-tocopherol (also an antioxidant and vitamin) antagonizes the pain relief effects of aspirin. This antagonism illustrates the specificity of the antioxidant-vitamin nutrient compounds which show an enhanced effect with analgesic drugs.

It should be understood that many modifications and variations can be made in the proportions and components used herein without departing from the spirit and scope of the invention, which is solely defined by the claims.

**Claims**

1. A composition, comprising an analgesic/anti-inflammatory drug and ß-carotene.

2. The composition of Claim 1 wherein the analgesic/anti-inflammatory drug is chosen from the group consisting of aspirin, acetaminophen, or mixtures thereof.

3. An enhanced analgesic/anti-inflammatory composition comprising ß-carotene and aspirin.

4. Process for manufacturing a composition, comprising an analgesic/anti inflammatory drug and ß-carotene characterized by mixing the active substances optionally together with usual auxiliaries and forming a suitable administration form.